# FASCICULE DE BREVET EUROPEEN

(11) **EP 3 015 129 B1**
(45) Date de publication et mention de la délivrance du brevet: **22.12.2021**
(21) Numéro de dépôt: 15191935.4
(22) Date de dépôt: 28.10.2015
(51) Int. Cl.: A61M 25/02, A61F 13/00

(54) **DISPOSITIF DE FIXATION D'UN ÉLÉMENT ALLONGÉ SUR LE CORPS D'UN PATIENT, NÉCESSAIRE DE TRAITEMENT ET PROCÉDÉ ASSOCIÉ**
VORRICHTUNG ZUR BEFESTIGUNG EINES LÄNGLICHEN ELEMENTS AUF DEM KÖRPER EINES PATIENTEN, INSTRUMENTENSET UND ENTSPRECHENDES VERFAHREN
DEVICE FOR SECURING AN ELONGATE MEMBER TO THE BODY OF A PATIENT, TREATMENT KIT AND ASSOCIATED METHOD

(30) Priorité: 28.10.2014 FR 1460355
(43) Date de publication de la demande: 04.05.2016
(73) Titulaire: Perouse Medical, 60173 Ivry le Temple (FR)
(72) Inventeur: JAOUANI, Benjamin, lahoucine, 94000 CRETEIL (FR)
(74) Mandataire: Lavoix

(56) Documents cités:
- WO-A1-2014/149668
- US-A- 3 826 254
- US-A- 4 838 878
- US-A1- 2008 200 880
- US-A1- 2012 226 237
- None

## Description

La présente invention concerne un dispositif de fixation d'un élément allongé sur le corps d'un patient, l'élément allongé étant destiné à être partiellement inséré dans le corps humain, le dispositif étant selon le préambule de la revendication 1. L'invention concerne également un nécessaire de traitement selon la revendication 13 et un procédé de fixation d'un élément allongé selon la revendication 15.

Certains éléments allongés destinés à être insérés dans le corps d'un patient permettent d'introduire ou de prélever des liquides dans le corps humain, par exemple par l'intermédiaire du réseau sanguin.

Ceci est le cas notamment des cathéters, ou plus spécialement encore des « PICC », c'est-à-dire des cathéters centraux insérés par voie périphérique selon le sigle anglais.

Ainsi, une première extrémité de l'élément allongé est introduite dans le corps humain, tandis qu'une deuxième extrémité est maintenue en dehors du corps humain, pour être reliée à un ou plusieurs tuyaux d'alimentation permettant le passage de fluide.

L'élément allongé est inséré dans le corps humain à partir d'un point de sortie, situé sur la peau du patient, par exemple au niveau d'un bras.

La première extrémité du cathéter est placée dans un site d'injection ou de prélèvement précis, par exemple au voisinage d'une valve cardiaque. La position de la première extrémité doit être maintenue de manière précise, afin d'assurer une distribution ou un prélèvement adéquat du produit, tout en évitant le risque de complications (par exemple une thrombose en cas de mauvais placement dans le réseau sanguin).

Lorsque le cathéter n'est pas utilisé, il doit être maintenu en position, afin de permettre au patient d'évoluer librement, sans risquer de déplacer la première extrémité.

De même, lorsque le cathéter est utilisé, la position de la première extrémité doit être maintenue, afin de garantir par exemple l'efficacité du traitement ou pour éviter les complications.

Il existe deux méthodes pour fixer la deuxième extrémité de l'élément allongé. La première méthode consiste à suturer l'extrémité du cathéter à la peau. Ceci assure que la fixation est efficace, mais constitue une étape traumatisante présentant des risques d'infection pour le patient et le soignant (en particulier, risque de piqure avec l'aiguille de suture).

Une deuxième méthode consiste à faire une fixation sans sutures, par exemple avec un simple pansement.

Des dispositifs à base de pansement ont été développés pour limiter le risque de mobilisation de l'élément allongé.

Les éléments allongés, comme les « PICC », sont généralement utilisés pendant de longues durées, et souvent en dehors de l'hôpital chez des patients mobiles. Il est donc nécessaire de changer régulièrement les pansements qui retiennent la deuxième extrémité de l'élément allongé à la peau du patient. Afin de diminuer les risques d'infection, les pansements doivent ainsi rester au maximum huit jours sur le patient.

Le retrait du pansement existant et la mise en place d'un nouveau pansement sont susceptibles de mobiliser l'élément allongé, ce qui peut être dommageable selon l'endroit où la première extrémité de l'élément allongé est insérée.

Par exemple, la première extrémité de l'élément allongé peut être insérée dans la veine cave supérieure. Un déplacement axial de l'élément allongé peut alors toucher le coeur ou provoquer des thromboses. De plus, un déplacement du cathéter peut perforer la veine dans laquelle il se trouve, ce qui peut engendrer des complications et des douleurs.

Par ailleurs, le simple retournement de la deuxième extrémité du cathéter, à l'extérieur du patient, pour la mise en place du pansement ou son retrait peut être désagréable pour le patient.

Pour pallier ce problème, il est connu de proposer des éléments allongés comportant, au niveau de leur deuxième extrémité, des ailettes qui sont fixées sur un support par deux clapets, associés chacun à une ailette. L'élément allongé est ainsi maintenu dans toutes les directions, et l'ensemble est fixé sur une bande adhésive. Un nécessaire de ce type est décrit dans le document WO 02/11786 A2.

Un tel nécessaire ne donne pas entière satisfaction. En effet, lors du changement de pansement, il est nécessaire de soulever les clapets retenant l'élément allongé, d'excentrer ou/et de replier l'élément allongé et de retirer le pansement afin de désinfecter la zone autour du point de sortie. Cette manœuvre mobilise significativement l'élément allongé.

De plus, le dispositif précité manque de simplicité, car les deux mains sont nécessaires pour procéder à l'installation et au retrait du dispositif. En outre, la prise qui permet l'ouverture des clapets, sur le bord inférieur, n'est pas clairement identifiée, et la prise à un autre endroit, sur le bord supérieur ou au milieu du clapet, rend difficile l'ouverture du clapet.

US 2008/200880, US 3 826 254 et US 4 838 878, ainsi que WO 2014/149668 A1 et US 2012/226237 A1 décrivent des pansements qui présentent un support de retenue au moins partiellement adhérent avec la peau du patient ou qui ne présentent pas de volet d'enserrement.

Un but de l'invention est de proposer un dispositif de fixation d'un élément allongé, qui limite encore plus les risques de mobilisation de l'élément allongé, tout en étant simple à utiliser à la fois pour la mise en place et pour le retrait du dispositif.

A cet effet, l'invention a pour objet un dispositif selon la revendication 1.

Le dispositif selon l'invention peut comprendre l'une ou plusieurs des caractéristiques des revendications 2 à 12 ou/et l'une des caractéristiques suivantes, prise(s) isolément ou suivant toute combinaison techniquement possible :
- la bande adhésive présente une région centrale située en regard du support de retenue, la ou chaque région externe faisant saillie latéralement par rapport à la région centrale et par rapport au support de retenue, une surface inférieure de la bande adhésive destinée à entrer en contact avec la peau du patient étant totalement non adhésive dans la région centrale, une surface supérieure de la bande adhésive portant le support de retenue.
- la surface inférieure de la paroi inférieure est propre à être appliquée sur la peau du patient en étant totalement non adhérente avec la peau du patient.
- la bande adhésive comprend au moins trois régions externes adhésives disjointes, avantageusement au moins quatre régions externes adhésives disjointes, les régions externes adhésives étant réparties autour du support de retenue, de part et d'autre par rapport à l'axe d'insertion.

L'invention a également pour objet un nécessaire de traitement selon la revendication 13.

Le nécessaire selon l'invention peut comprendre la caractéristique de la revendication 14.

L'invention a également pour objet un procédé de fixation selon la revendication 15.

Le procédé selon l'invention peut comprendre les caractéristiques de la revendication 16.

L'invention sera mieux comprise à la lecture de la description qui va suivre, donnée uniquement à titre d'exemple, et faite en se référant aux dessins annexés, sur lesquels :
- la figure 1 est une vue du dessus d'un premier nécessaire de traitement selon l'invention comportant un élément allongé, un dispositif de fixation de l'élément allongé et un pansement supérieur destiné à recouvrir l'ensemble ;
- la figure 2 est une vue de dessus du nécessaire après sa mise en place ;
- la figure 3 est une vue de côté du support de retenue dans le dispositif de fixation de la figure 1, comportant dans cet exemple une paroi inférieure, un volet d'enserrement et un volet de fixation ;
- la figure 4 est une vue de dessus du support de retenue de la figure 3 lorsqu'il occupe sa position dépliée ;
- la figure 5 est une vue de côté du mécanisme de fixation sélective du volet de fixation sur le volet d'enserrement du support de retenue de la figure 3 ;
- la figure 6 est une vue en perspective d'un deuxième dispositif de fixation selon l'invention lors de son montage ;
- la figure 7 est une vue de côté d'une variante de système d'accroche de l'élément allongé pour un dispositif de fixation comportant des picots ;
- la figure 8 est une représentation schématique de la bande adhésive selon une deuxième réalisation ;
- la figure 9 est une vue de côté d'un élément de contact non glissant fixé contre la paroi inférieure ;
- la figure 10 est une vue de dessus du support de retenue en position dépliée, en présence d'un deuxième mécanisme de fixation sélective selon l'invention, et
- la figure 11 est une vue de dessus du nécessaire après sa mise en place, dans lequel le pansement supérieur comprend un volet de maintien d'une extrémité de l'élément allongé.

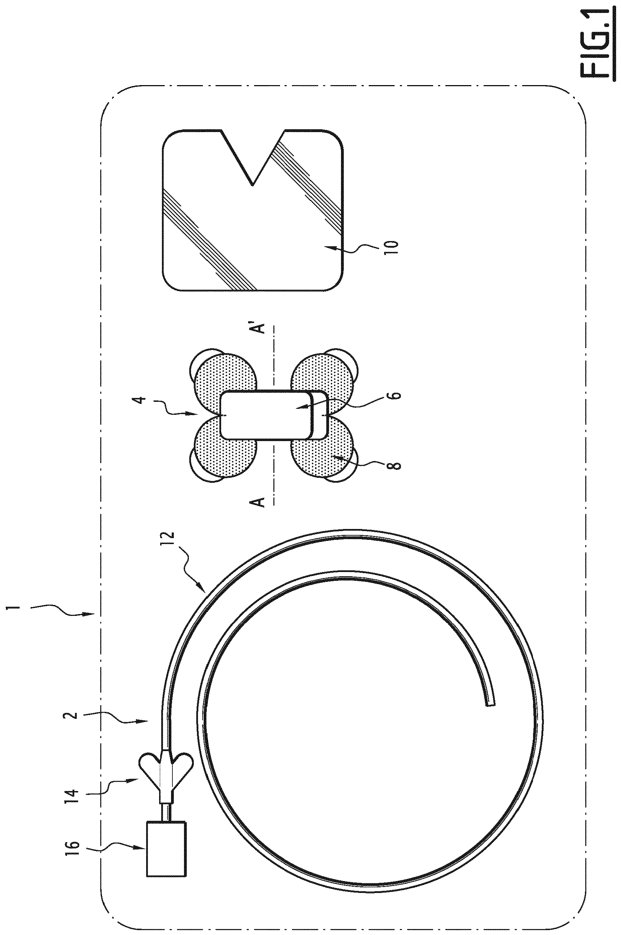

Un premier nécessaire 1 de traitement selon l'invention est représenté sur la figure 1. Le nécessaire 1 comprend un élément allongé 2, destiné à être introduit au moins partiellement dans le corps d'un patient, et un premier dispositif 4 de fixation de l'élément allongé 2 sur le corps du patient.

Le dispositif 4 comprend, selon l'invention, un support de retenue 6, et une bande adhésive 8 portant le support de retenue 6 pour le fixer sur une surface corporelle du patient.

Le nécessaire 1 comporte en outre avantageusement un pansement supérieur 10.

L'élément allongé 2 comporte dans cet exemple un tuyau souple 12, et une embase de manipulation 14.

Le tuyau souple 12 s'étend entre une première extrémité destinée à être introduite dans le corps du patient, par exemple dans une veine, et une deuxième extrémité destinée à être maintenue en dehors du corps du patient et à être reçue dans le support de retenue 6 du dispositif 4.

La longueur du tuyau souple 12 est par exemple comprise entre 5 cm et 60 cm, et son diamètre intérieur est par exemple compris entre 0,30 mm et 8 mm.

L'embase de manipulation 14 fait saillie à l'extérieur du tuyau souple 12 au voisinage de la deuxième extrémité. Elle est maintenue en permanence à l'extérieur du corps du patient.

Le tuyau souple 12 présente, à sa deuxième extrémité, au moins un connecteur 16 de raccordement fluidique de l'élément allongé 2 à au moins un ensemble de transfert de fluide depuis l'extérieur du corps du patient vers l'intérieur du corps du patient (ou inversement).

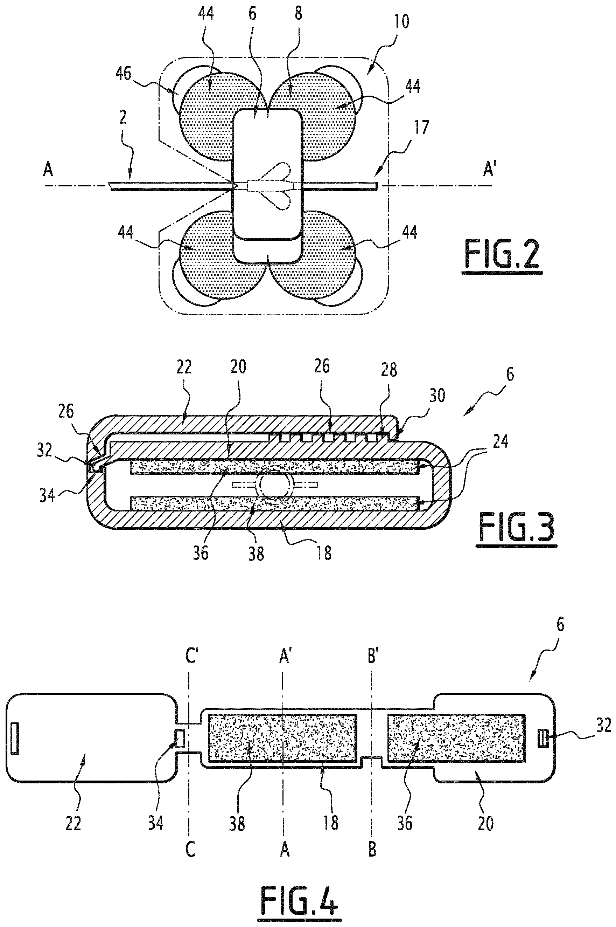

La première extrémité du tuyau souple 12 est avantageusement introduite dans le corps du patient par l'intermédiaire d'un point de sortie 17 dans la peau du patient (visible sur la figure 2). Elle est amenée jusqu'à un site d'injection ou/et de prélèvement de fluide dans le patient, avantageusement à travers son système circulatoire.

L'ensemble formé par le tuyau souple 12 et l'embase de manipulation 14 constitue notamment un cathéter de type cathéter central inséré par voie périphérique ou « PICC ».

Comme indiqué plus haut, le dispositif 4 comporte le support de retenue 6 fixé sur la bande adhésive 8.

La figure 3 représente une vue de côté du support de retenue 6. Le support de retenue 6 est destiné à maintenir de manière réversible une partie de l'élément allongé 2 faisant saillie hors du corps du patient à partir du point de sortie 17.

Le support de retenue 6 est formé par une pince qui comprend une paroi inférieure 18 fixée sur la bande adhésive 8, un volet d'enserrement 20 mobile par rapport à la paroi inférieure 18 entre une position ouverte et une position d'enserrement de l'élément allongé 2, et avantageusement un volet de fixation 22, mobile par rapport au volet d'enserrement 20 entre une position de repos et une position de maintien du volet d'enserrement 20 dans la position d'enserrement de l'élément allongé 2.

Le support de retenue 6 comporte en outre un ensemble de calage 24 de l'élément allongé 2, interposé entre la paroi inférieure 18 et le volet d'enserrement 20 dans la position d'enserrement, et un ensemble de fixation 26 réglable du volet 22 de fixation dans sa position de maintien.

La paroi inférieure 18 du support de retenue 6 est par exemple de forme rectangulaire. La paroi inférieure 18 comprend avantageusement une ou deux encoches opposées l'une à l'autre, comprises sur les bords longitudinaux de la paroi inférieure 18, afin de permettre l'utilisation d'un pansement antimicrobien et/ou d'hémostase. Ce type de pansement est par exemple décrit dans le brevet US 8 579 863 B2.

Elle s'étend transversalement par rapport à un axe A-A' d'introduction et de maintien de l'élément allongé 2 dans le support de retenue 6.

La paroi inférieure 18 est rigide en flexion, au sens qu'elle est plus rigide en flexion que la bande adhésive 8.

Ainsi, de préférence, lorsque les bords latéraux de la paroi inférieure 18 sont saisis entre les doigts d'un utilisateur, ils ne peuvent pas être fléchis l'un vers l'autre manuellement, ou ils peuvent être fléchis l'un vers l'autre, sans pouvoir entrer en contact l'un avec l'autre sans déformation irréversible de la paroi inférieure 18.

La paroi inférieure 18 est par exemple réalisée à base d'un matériau thermoplastique tel que du PE, de l'ABS, du MABS, du PU, du silicone, un fluoropolymère, du TPE, du PP ou du PC.

La paroi inférieure 18 présente une surface inférieure fixée en partie ou en totalité sur une partie centrale de la bande adhésive 8. La surface inférieure de la paroi inférieure 18 est non collante et atraumatique.

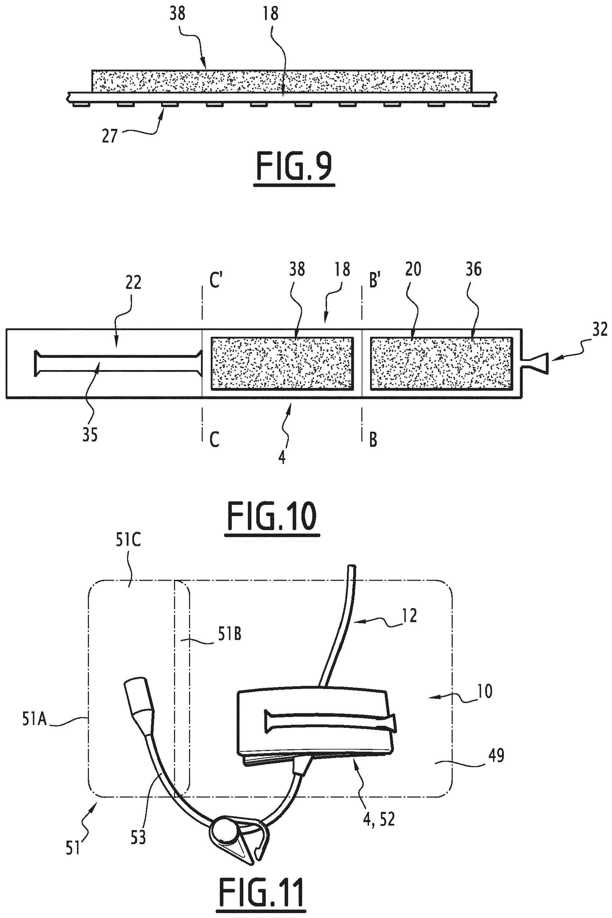

En variante visible sur la figure 9, un élément de contact non glissant 27 est fixé sur la surface inférieure de la paroi inférieure 18. L'élément de contact non glissant 27 est par exemple une mousse ou une plaque faite en silicone, en PU ou en TPE. Dans l'exemple représenté sur la figure 9, l'élément de contact non glissant 27 comporte plusieurs bandelettes disposées parallèlement entre elles, perpendiculairement à l'axe A-A'. Les bandelettes définissent entre elles des canaux permettant la circulation de l'air, ce qui améliore la respiration de la peau lorsque le dispositif 4 est installé sur la peau du patient.

Avantageusement, les bandelettes sont en mousse.

Comme on le verra plus bas, selon l'invention, le support de retenue 6 est non adhérent à la peau du patient, située dans l'axe A-A' en regard de toute la paroi inférieure 18.

Le volet d'enserrement 20 est également rigide en flexion, comme défini précédemment. Il présente une longueur sensiblement égale à celle de la paroi inférieure 18, comme visible sur la figure 4.

Le volet d'enserrement 20 est articulé sur un premier bord latéral de la paroi inférieure 18, autour d'un axe d'articulation B-B' sensiblement parallèle à l'axe d'introduction et de maintien A-A'.

Il est propre à se rabattre sur la paroi inférieure 18, par pivotement autour de l'axe B-B' entre la position ouverte, visible sur la figure 4, et la position d'enserrement de l'élément allongé 2, visible sur la figure 3.

Dans la position ouverte, le volet d'enserrement 20 est décalé latéralement par rapport à la paroi inférieure 18. L'élément allongé 2 est propre à être introduit sans pivotement autour du point de sortie 17, en insérant la paroi inférieure 18 sous l'élément allongé 2 suivant un mouvement transversal par rapport à l'axe A-A', ou suivant un mouvement axial par rapport à l'axe A-A'.

Dans la position d'enserrement, le volet d'enserrement 20 a été pivoté autour de l'axe B-B', pour le placer en regard de la paroi inférieure 18, au-dessus de celle-ci. L'élément allongé 2 est propre à être calé entre le volet d'enserrement 20 et la paroi inférieure 18 dans cette position.

Le volet de fixation 22 est articulé sur la paroi inférieure 18 le long d'un deuxième bord latéral de la paroi inférieure 18 autour d'un axe de pivotement C-C' parallèle à l'axe A-A' et à l'axe B-B', et opposé au premier bord.

Il présente une longueur sensiblement identique à celle du volet d'enserrement 20 et à celle de la paroi inférieure 18.

Il est propre à se rabattre sur le volet d'enserrement dans sa position d'enserrement, par pivotement autour de l'axe C-C' entre la position ouverte, visible sur la figure 4, et la position de maintien du volet d'enserrement, visible figure 3.

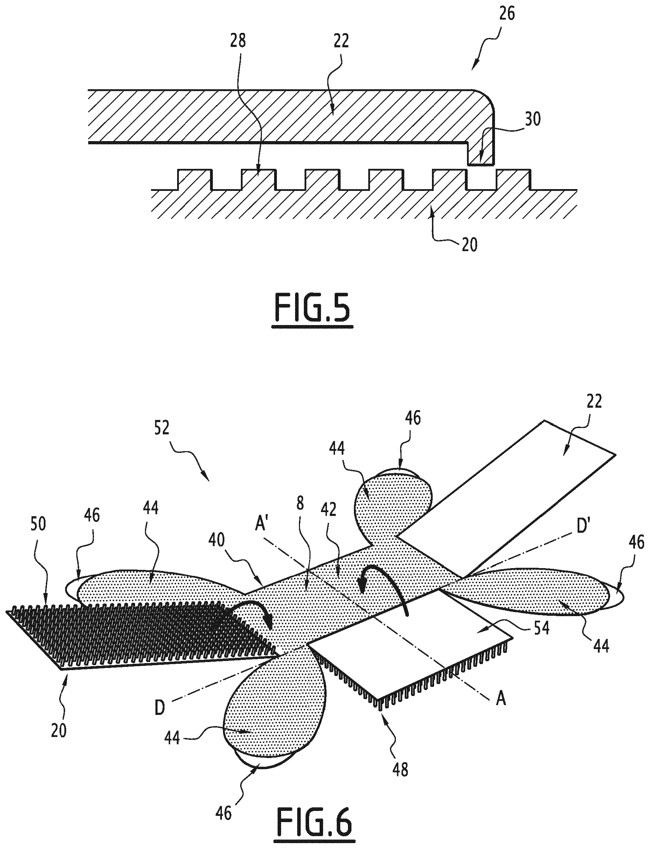

Comme illustré sur la figure 5, une région d'extrémité du volet de fixation 22 est apte à se fixer de manière sélective en longueur sur le volet d'enserrement 20 par l'intermédiaire de l'ensemble de fixation 26.

Le volet de fixation 22 est également rigide en flexion, comme défini précédemment.

En référence à la figure 5, l'ensemble de fixation 26 comporte par exemple des créneaux 28 formés dans une surface supérieure du volet d'enserrement 20, et une saillie d'accrochage 30 formée dans une surface inférieure du volet de fixation 22.

Les créneaux 28 sont espacés transversalement par rapport à l'axe A-A', le long du volet d'enserrement 20. Ils définissent une pluralité de zones d'accrochage de la saillie d'accrochage 30 espacées transversalement par rapport à l'axe A-A'.

La fixation sélective de la saillie d'accrochage 30 dans l'un des créneaux 28 adapte le serrage de l'élément allongé 2 en fonction de sa taille ou de sa forme, et constitue, avec le volet d'enserrement 20, un maintien supplémentaire de l'élément allongé 2.

Avantageusement, l'ensemble de fixation 26 comporte en outre un élément de fixation réversible 32 du volet d'enserrement sur la paroi inférieure, et un élément de réception 34 de l'élément de fixation réversible 32.

L'élément de fixation réversible 32 est par exemple formé par un crochet faisant saillie transversalement à l'extrémité libre du volet d'enserrement 20. L'élément de réception 34 est constitué par un logement de réception du crochet ménagé dans la paroi inférieure 18 au voisinage de l'axe C-C'.

Dans une première variante, l'ensemble de fixation 26 comprend une première bande en velcro (non représentée) et une seconde bande en velcro (non représentée). La première bande en velcro est fixée sur une surface supérieure du volet d'enserrement 20. La deuxième bande en velcro est fixée sur une surface inférieure du volet de fixation 22.

Dans une variante, une combinaison de crochet et de bande en velcro forment l'ensemble de fixation.

Dans une seconde variante représentée sur la figure 10, l'ensemble de fixation 26 comprend une rainure 35 traversant le volet de fixation 22, dans laquelle peut s'engager l'élément de fixation réversible 32.

La rainure 35 s'étend suivant un axe longitudinal du volet de fixation 22, ici perpendiculairement à l'axe A-A'.

La largeur de la rainure 35 est supérieure à la largeur de l'élément de fixation réversible 32.

Selon un mode de réalisation, la largeur de la rainure 35 est constante sur toute la longueur de la rainure 35.

Selon un autre mode de réalisation, la largeur de la rainure 35 décroît lorsqu'elle se rapproche de l'axe C-C'.

Dans la position ouverte, l'élément de fixation réversible 32 est propre à être introduit dans la rainure 35 à une extrémité de la rainure. Il est propre à coulisser vers une extrémité opposée de la rainure lors du passage dans la position d'enserrement.

L'ensemble de calage 24 est destiné, lorsque le volet d'enserrement 20 occupe sa position d'enserrement, à maintenir en place l'élément allongé 2 entre la paroi inférieure 18 et le volet d'enserrement 20.

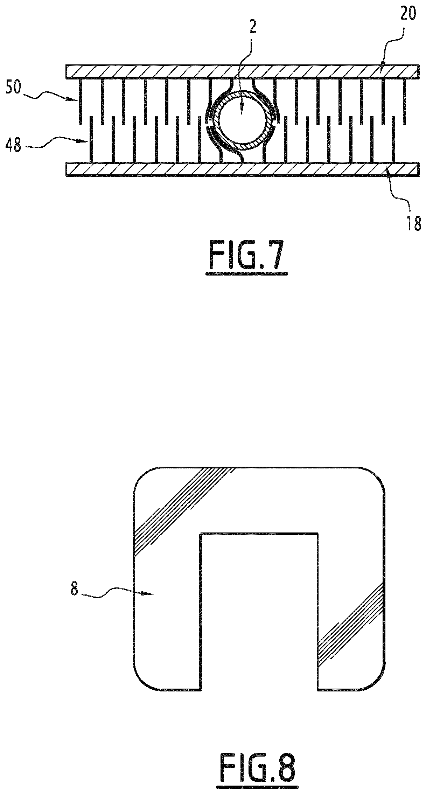

L'ensemble de calage 24 comporte par exemple des éléments en matériau déformable élastiquement, par exemple des blocs de mousse, comme illustré sur la figure 3, ou des picots, comme illustré par la figure 7.

En variante, l'ensemble de calage 24 est réalisé avantageusement à base de mousse épaisse relativement dure, moins dure que le volet d'enserrement 20, et comporte une découpe universelle permettant de placer n'importe quelle embase de manipulation 14. L'ensemble de calage 24 renforce le maintien de l'élément allongé 2 grâce à sa hauteur et à la forme de la découpe.

Dans l'exemple représenté par la figure 3 et par la figure 4, l'ensemble de calage 24 comporte au moins un premier élément en matériau déformable 36 porté par une surface supérieure de la paroi inférieure 18, et au moins un deuxième élément en matériau déformable 38, porté par une surface inférieure du volet d'enserrement 20.

La bande adhésive 8 est destinée à fixer le support de retenue 6 sur la peau du patient. Elle définit une surface inférieure 40 destinée à entrer en contact avec la peau du patient et une surface supérieure 42, portant le support de retenue 6. Dans cet exemple, le support 6 est fixé sur la surface supérieure.

Dans l'exemple représenté sur la figure 2, la bande adhésive 8 comporte une région centrale située en regard du support de retenue 6 et au moins une région externe 44 faisant saillie latéralement par rapport à la région centrale et par rapport au support de retenue 6.

Dans la région centrale, la surface inférieure 40 est au moins partiellement non adhésive, et de préférence totalement non adhésive. Ainsi, lors du retrait du dispositif de fixation 4, la région centrale portant le support de retenue 6 est apte à être retirée par coulissement sur la peau, sans avoir à soulever significativement l'élément allongé 2, et en tout état de cause, sans avoir à le retourner. De plus, il n'y a pas de risque de décollement du pansement 8 par rapport au support de retenue 6 en cas de transpiration ou de suintement.

De préférence, la bande adhésive 8 comporte au moins deux régions externes 44 opposées, situées de part et d'autre de l'axe A-A', en délimitant entre elles, le long de l'axe A-A', la région centrale.

Avantageusement, dans l'exemple représenté sur la figure 1, la bande adhésive 8 présente une forme de trèfle à quatre feuilles, avec quatre régions externes 44 réparties angulairement autour de la région centrale, en formant des pétales disjoints.

Deux régions externes 44 sont situées d'un côté de l'axe A-A', et deux autres régions externes 44 sont situées d'un autre côté de l'axe A-A'.

Les régions externes 44 situées de part et d'autre de l'axe A-A' délimitent entre elles un espace intermédiaire s'étendant suivant l'axe central A-A' dépourvu de région externe 44.

Chaque région externe 44 est située à l'écart du support de retenue 6 et est donc accessible à l'utilisateur, sans avoir à soulever le support de retenue 6, notamment pour les décoller de la peau du patient.

Dans chaque région externe 44, la surface inférieure 40 est au moins en partie adhésive, de préférence est totalement adhésive. Elle est avantageusement munie d'une languette de protection 46 amovible fixée en-dessous de la partie adhésive de la surface inférieure 40. De préférence, la languette de protection 46 fait saillie partiellement vers l'extérieur par rapport à la bande adhésive 8, pour permettre le retrait aisé de la languette de protection 46, afin de découvrir la partie adhésive de la surface inférieure 40.

La surface supérieure 42 porte le support de retenue 6. La région centrale de la surface supérieure 42 retient le support de retenue 6 par un adhésif, les autres parties de la surface supérieure 42 étant dépourvues d'adhésif.

La bande adhésive 8 est déformable. Elle est moins rigide en flexion que la paroi inférieure 18.

Ainsi, de préférence, lorsque les bords latéraux de la bande adhésive 8 sont saisis entre les doigts d'un utilisateur, ils peuvent être fléchis l'un vers l'autre manuellement, jusqu'à venir au contact l'un de l'autre, de manière réversible.

Le pansement supérieur 10 sert à maintenir l'ensemble constitué par le dispositif 4 retenant l'élément allongé 2, sur la peau du patient. Il constitue un moyen de fixation supplémentaire de l'élément allongé 2.

Le pansement supérieur 10 est par exemple de forme rectangulaire (figure 1 et 2), avec une encoche en forme de V au milieu de l'un de ses côtés, de manière à avoir un meilleur maintien de l'ensemble constitué par l'élément allongé 2, le support de retenue 6 et la bande adhésive 8.

L'encoche en forme de V sur le pansement supérieur 10 est située selon l'axe A-A' du côté opposé au point de sortie 17. Ainsi, il est possible de visualiser le point de sortie 17, même lorsque le dispositif 4 est installé sur la peau du patient.

Dans une variante représentée sur la figure 11, le pansement supérieur 10 comporte une feuille de base 49, destinée à coiffer le dispositif 4 et un volet de maintien 51 de l'extrémité 53 de l'élément allongé 2, articulé sur la feuille de base 49 par un premier bord 51A.

La surface interne de la feuille de base 49 est au moins partiellement adhésive pour coller sur la peau du patient. La surface externe de la feuille de base est avantageusement non adhésive.

Le volet de maintien 51 est propre à se rabattre sur le pansement supérieur 10.

La surface supérieure du volet de maintien 51 est non adhésive. La surface inférieure du volet de maintien 51 est non adhésive dans une zone centrale 51C du volet de maintien 51. Elle est adhésive suivant le premier bord 51A et suivant un deuxième bord 51B du volet de maintien 51.

La surface interne du volet de maintien 51 et la surface externe de la feuille 49 définissent entre elles un espace intermédiaire d'immobilisation de l'extrémité 53 de l'élément allongé 2.

Ainsi, quelle que soit la longueur du tuyau souple 12, il est possible d'amener l'extrémité 53 du tuyau souple 12 en appui sur la surface externe de la feuille 49, après mise en place de la feuille 49 sur le dispositif 4, en laissant le volet de maintien ouvert. L'extrémité 53 est alors fixée sur la surface extérieure en rabattant le volet de maintien 51.

Le fonctionnement du nécessaire de fixation 1 va maintenant être décrit.

Initialement, l'élément allongé 2 a été mis en place dans le corps du patient. Il présente une première extrémité située à un site d'injection, un tronçon interne s'étendant dans le patient jusqu'au point de sortie 17, et un tronçon externe faisant saillie hors du corps du patient, sur la peau du patient, jusqu'à une deuxième extrémité le long d'un axe A-A'.

Un nécessaire de fixation 1 est alors fourni. L'opérateur saisit le dispositif 4 et place le volet d'enserrement 20 dans sa position ouverte, et le volet de fixation 22 dans sa position de repos.

Il oriente ensuite le support de retenue 6 suivant un axe perpendiculaire à l'axe A-A' de l'élément allongé 2. Le volet d'enserrement 20 est dans sa position ouverte. Le support de retenue 6 peut être placé au choix dans un sens ou dans l'autre le long de l'axe perpendiculaire à l'axe A-A'.

Il glisse alors la bande adhésive 8 munie des languettes de protection 46 et la paroi inférieure 18 sous l'élément allongé 2, soit latéralement, soit axialement en maintenant en position l'élément allongé 2.

Le soulèvement de l'élément allongé 2 est minimal, ce qui limite sa mobilisation. En aucun cas il n'est nécessaire de retourner l'élément allongé 2 autour du point de sortie 17. La région centrale étant dépourvue d'adhésif, le support coulisse facilement sur la peau sous l'élément allongé.

Le volet d'enserrement 20 est ensuite pivoté autour de l'axe B-B' pour le passer dans sa position d'enserrement, et saisir transversalement l'élément allongé 2, dans l'ensemble de calage 24.

L'élément de fixation réversible 32 situé à l'extrémité du volet d'enserrement 20 est introduit dans l'élément de réception 34 ménagé dans la paroi inférieure 18.

Puis, le volet de fixation 22 est pivoté autour de l'axe C-C' pour être rabattu sur le volet d'enserrement 20. En fonction de la hauteur de l'élément allongé 2 enserré entre le volet d'enserrement 20 et la paroi inférieure 18, l'opérateur introduit la saillie d'accrochage 30 dans un créneau 28 adapté.

L'élément allongé 2 est alors maintenu en position dans le support de retenue 6.

Les languettes de protection 46 des régions externes 44 de la bande adhésive 8 sont ensuite retirées. La bande adhésive 8 est fixée solidement sur la peau du patient.

Ensuite, le pansement supérieur 10 est placé sur le dispositif 4 pour le protéger.

Lors du retrait du dispositif, le pansement supérieur 10 est d'abord retiré.

Les régions externes 44 de la bande adhésive 8 sont ensuite décollées. Les régions externes 44 sont ensuite avantageusement recollées sur elles-mêmes pour éviter de se recoller sur la peau. Le volet de fixation 22 et le volet d'enserrement 20 sont repassés respectivement dans leur position de repos et dans leur position ouverte.

Selon l'invention, toute la région centrale de la bande adhésive 8 située en regard du support de retenue 6 étant non adhérente avec la peau du patient, le dispositif 4 peut à nouveau être glissé sous l'élément allongé 2 entre l'élément allongé 2 et la peau du patient, sans avoir à soulever l'élément allongé 2.

Contrairement aux dispositifs connus de l'état de la technique, la mise en place et le retrait du dispositif 4 ne nécessitent pas un soulèvement significatif de l'élément allongé 2, grâce à l'utilisation d'un support de retenue 6 formé d'une pince, et grâce à la non adhérence du dispositif 4 sur la peau du patient en regard du support de retenue 6.

Ainsi, le dispositif 4 peut être mis en place et retiré avec une mobilisation minimale de l'élément allongé 2.

Lors du retrait du dispositif de fixation 4, une main de l'utilisateur maintient l'élément allongé 2 et l'autre main manipule le dispositif de fixation 4.

Lorsque le retrait du dispositif de fixation 4 est effectué pour installer un nouveau dispositif de fixation 4, il n'est pas nécessaire de nettoyer à l'alcool la portion de l'élément allongé 2 précédemment maintenue par le dispositif de fixation 4, car aucune portion du dispositif de fixation 4 retenant l'élément allongé 2 n'est collante. De plus, le dispositif de fixation 4 est propre à être utilisé indifféremment dans un sens axial ou dans l'autre, ce qui facilite le travail du praticien.

Dans une variante, l'ensemble de calage 24 de l'élément allongé 2 comporte deux jeux de picots opposés comme représenté sur la figure 7. Un premier jeu de picots 48 fait saillie vers le haut à partir de la surface supérieure de la paroi inférieure 18, et un deuxième jeu de picots 50 fait saillie vers le bas à partir de la surface inférieure du volet d'enserrement 20 pour s'insérer entre les picots du premier jeu de picots 48 dans la position d'enserrement.

Un tel ensemble de calage 24 présente l'avantage de maintenir et de s'adapter à tout type d'élément allongé 2, quelle que soit sa forme ou son diamètre.

La figure 6 représente de manière schématique un deuxième dispositif 52 de fixation selon l'invention.

À la différence du premier dispositif 4, la bande adhésive 8 est en contact avec la peau du patient au niveau de régions externes 44, mais pas de sa région centrale.

La région centrale de la bande adhésive 8 chevauche la paroi inférieure 18, pour se fixer sur celle-ci. La surface supérieure de la paroi inférieure 18 est appliquée sur la surface inférieure 40 de la bande adhésive 8, au niveau de la région centrale.

La surface inférieure de la paroi inférieure 18 est appliquée contre la peau du patient. Cette surface inférieure est dépourvue d'adhésif, et est non adhérente avec la peau du patient. Comme décrit précédemment, un élément de contact non glissant 27 est avantageusement fixé sur la surface inférieure de la paroi inférieure 18.

Avantageusement, le support de retenue 6 possède, outre la paroi inférieure 18, le volet d'enserrement 20 et le volet de fixation 22, un volet de rabattement 54 sur la région centrale de la bande adhésive 8.

Le volet de rabattement 54 est de longueur inférieure à celle de la paroi inférieure 18. Il est articulé sur la paroi inférieure 18, suivant un bord longitudinal autour d'un axe D-D' sensiblement perpendiculaire à l'axe A-A'.

Lors du montage du dispositif 6, le volet de rabattement 54 est propre à se replier sur la partie centrale de la bande adhésive 8 au niveau de la paroi inférieure 18, entre une position ouverte et une position repliée, dans laquelle il couvre la région centrale de la bande adhésive 8.

Avantageusement, comme illustré par la figure 6, le volet de rabattement 54 présente sur sa face supérieure un premier jeu de picots 48.

Le volet d'enserrement 20 présente sur sa face inférieure un deuxième jeu de picots 50, de sorte que, en se repliant sur le volet d'enserrement 20, les deux jeux de picots précédemment définis s'insèrent l'un dans l'autre.

Lorsque le volet de rabattement 54 et le volet d'enserrement 20 sont en position repliée, l'élément allongé 2 est ainsi maintenu entre les deux jeux de picots 48 et 50.

Lors du montage du dispositif 6, la région centrale de la bande adhésive 8 est fixée sur le support de retenue 6.

Le volet de rabattement 54 est maintenu dans sa position ouverte. La bande adhésive 8 est amenée en regard de la paroi inférieure 18. La surface supérieure de la paroi inférieure 18 du support de retenue 6 est alors fixée par collage à la région centrale de la surface inférieure 40 de la bande adhésive 8.

Puis, le volet de rabattement 54 est placé en position repliée, par pivotement autour de l'axe D-D' avec un premier jeu de picots 48 sur sa surface supérieure.

Lors de l'utilisation du dispositif 6, l'élément allongé 2 est ensuite positionné sur le volet de rabattement 30, puis est maintenu par le volet d'enserrement 20 en position d'enserrement, comme décrit précédemment.

Avantageusement, la paroi inférieure 18, le volet d'enserrement 20, le volet de fixation, le volet de rabattement et les deux jeux de picots sont formés d'un seul tenant en étant venus de matière, par exemple à base d'une matière thermoplastique telle que du PE, de l'ABS, du MABS, du PU, du silicone, un fluoropolymère, du TPE, du PP, ou du PC.

Le deuxième dispositif selon l'invention 6 est donc particulièrement simple et peu coûteux à fabriquer.

Dans une variante, illustrée par la figure 8, la bande adhésive 8 présente une forme en U. Elle présente ainsi des branches parallèles formant des régions externes adhésives raccordées entre elles par une branche centrale.

Le support de retenue 6 est fixé à chacune de ses extrémités sur les branches parallèles du U.

La surface inférieure du support de retenue 6 disposée entre les branches parallèles de la bande adhésive 8 et n'est pas adhérente à la peau du patient.

Grâce à l'invention qui vient d'être décrite, il est possible de visualiser le point de sortie 17 de l'élément allongé 2, même en cas de recouvrement avec un pansement supérieur 10 opaque.

Le dispositif de fixation 4, 52 est utilisable quelle que soit la longueur externalisée de l'élément allongé 2.

La mobilisation de l'élément allongé 2 est minimale lors de la pose et du retrait, et le dispositif 4, 52 est ni invasif, ni traumatique, en particulier grâce à la présence d'une paroi inférieure 18 totalement non adhésive.

Il résulte directement et sans ambigüité de ce qui précède que l'élément allongé 2 est maintenu entre le volet d'enserrement 20 et la paroi inférieure 18 dans la position d'enserrement, exclusivement par coopération mécanique. Aucun adhésif n'est disposé au contact de l'élément allongé 2 entre l'élément allongé 2 et le volet d'enserrement 20, ni entre l'élément allongé 2 et la paroi inférieure 18.

Lorsque le volet d'enserrement 20 occupe sa position ouverte, le support de retenue 6 est déplaçable librement par rapport à l'élément allongé 2, sans avoir à décoller l'élément allongé 2 du support de retenue 6.

## Revendications

1. Dispositif (4 ; 52) de fixation d'un élément allongé (2) sur le corps d'un patient, l'élément allongé (2) étant destiné à être partiellement inséré dans le corps du patient, le dispositif comportant :
- un support de retenue (6) de l'élément allongé (2), comportant au moins une paroi inférieure (18) destinée à être placée entre l'élément allongé (2) et le corps du patient
- une bande adhésive (8) portant le support de retenue (6), et destinée à être appliquée sur la peau du patient, la bande adhésive (8) définissant au moins une région du dispositif de fixation (4) déportée par rapport à la paroi inférieure (18) destinée à adhérer à la peau du patient
au moins une région du dispositif de fixation (4) située sous la paroi inférieure (18) suivant un axe (A-A') d'insertion de l'élément allongé (2) dans le support de retenue (6) est destinée à être non adhérente à la peau du patient lorsque le dispositif de fixation (4) est fixé sur la peau du patient,
le support de retenue (6) comprenant un volet d'enserrement (20) propre à être manœuvré par rapport à la paroi inférieure (18) entre une position ouverte et une position d'enserrement de l'élément allongé (2), dans laquelle l'élément allongé (2) est destiné à être maintenu entre la paroi inférieure (18) et le volet d'enserrement (20),
**caractérisé en ce que** toute la région du dispositif de fixation (4) située sous la paroi inférieure (18) du support de retenue (6) est destinée à être non adhérente avec la peau du patient.

2. Dispositif (4 ; 52) selon la revendication 1, dans lequel le volet d'enserrement (20) est plus rigide en flexion que la bande adhésive (8).

3. Dispositif (4 ; 52) selon l'une quelconque des revendications 1 à 2, dans lequel le support de retenue (6) comprend un volet de fixation (22), propre à se rabattre sur le volet d'enserrement (20) pour maintenir en position le volet d'enserrement (20),
le volet de fixation (22) étant avantageusement plus rigide en flexion que la bande adhésive (8).

4. Dispositif (4 ; 52) selon la revendication 3, comprenant un ensemble de fixation (26) sélective du volet de fixation (22) sur le volet d'enserrement (20).

5. Dispositif (4 ; 52) selon l'une quelconque des revendications précédentes, dans lequel le support de retenue (6) comprend au moins un ensemble de calage (24) de l'élément allongé (2), destiné à être interposé entre la paroi inférieure (18) et le volet d'enserrement (20) dans la position d'enserrement,
l'ensemble de calage (24) comportant avantageusement au moins un élément en un matériau déformable (36) élastiquement faisant saillie à partir du volet d'enserrement (20) et/ou de la paroi inférieure (18).

6. Dispositif (4) selon la revendication 5, dans lequel l'ensemble de calage (24) comprend un premier jeu de picots (48) faisant saillie à partir du volet d'enserrement (20) et un deuxième jeu de picots (50) faisant saillie à partir de la paroi inférieure (18).

7. Dispositif (4 ; 52) selon l'une quelconque des revendications précédentes, dans lequel la bande adhésive (8) présente au moins une région externe (44) adhésive destinée à entrer en contact avec le corps du patient, la ou chaque région externe (44) adhésive étant déportée par rapport au support de retenue (6) et avantageusement par rapport à un point de sortie (17) de l'élément allongé (2) hors du patient.

8. Dispositif (4 ; 52) selon l'une quelconque des revendications précédentes, dans lequel la bande adhésive (8) comprend au moins deux régions externes (44) adhésives situées de part et d'autre du support de retenue (6) par rapport à l'axe d'insertion.

9. Dispositif (4) selon l'une quelconque des revendications précédentes, dans lequel la bande adhésive (8) comporte une surface inférieure (40) et une surface supérieure (42), le support de retenue (6) étant fixé sur la surface supérieure (42), avantageusement par collage.

10. Dispositif (52) selon l'une quelconque des revendications 1 à 9, dans lequel la bande adhésive (8) comporte une surface inférieure (40) et une surface supérieure (42), au moins une région centrale de la bande adhésive (8) étant fixée sur une surface supérieure de la paroi inférieure (18), la région centrale chevauchant la paroi inférieure (18).

11. Dispositif (52) selon la revendication 10, dans lequel au moins un élément de contact non glissant (27) est fixé sur la surface inférieure de la paroi inférieure (18).

12. Dispositif (52) selon l'une quelconque des revendications précédentes, dans lequel le support de retenue (6) comporte un volet de rabattement (54), le volet de rabattement (54) étant appliqué sur la région centrale de la bande adhésive (8).

13. Nécessaire de traitement (1) comprenant un dispositif (4; 52) selon l'une quelconque des revendications précédentes et un élément allongé (2) destiné à être partiellement inséré dans le corps du patient, l'élément allongé (2) étant avantageusement choisi parmi un cathéter, notamment un cathéter central à insertion périphérique, un cathéter veineux central, un cathéter de dialyse, un cathéter urinaire, une sonde nasogastrique, un cathéter péritonéal, un cathéter de Foley, un cathéter de drainage, une sonde biliaire, une sonde de néphrostomie, une sonde de gastrostomie, un cathéter épidural, un cathéter artériel, une sonde de pyélostomie, un cathéter péri-nerveux et/ou une tubulure ou le corps d'une aiguille Huber.

14. Nécessaire de traitement (1) selon la revendication 13, comprenant en outre un pansement supérieur (10) destiné à recouvrir le dispositif (4; 52), le pansement supérieur (10) comprenant une feuille de base (49) coiffant le dispositif (4) et un volet de maintien (51) propre à se rabattre sur la feuille de base (49) et définissant un espace d'immobilisation d'une extrémité (53) de l'élément allongé (2).

15. Procédé de fixation d'un élément allongé (2) sur le corps d'un patient, mise en oeuvre après l'insertion partielle de l'élément allongé (2) dans le corps du patient, le procédé comprenant les étapes suivantes :
- fourniture d'un dispositif (4 ; 52) selon les revendications 1 à 12 ;
- placement du volet d'enserrement (20) dans sa position ouverte et insertion de l'élément allongé (2) sur la paroi inférieure (18) en glissant la paroi inférieure (18) sous l'élément allongé (2), sans retourner l'élément allongé (2) ;
- passage du volet d'enserrement (20) dans sa position d'enserrement ;
- fixation d'au moins une région externe de la bande adhésive (8) sur la peau du patient, au moins une région du dispositif (4 ; 52) située sous la paroi inférieure (18) suivant un axe d'insertion de l'élément allongé dans le support étant non adhérente à la peau du patient.

16. Procédé selon la revendication 15, comportant les étapes suivantes :
- décollage de chaque région externe (44) à l'écart de la peau du patient, en maintenant le volet d'enserrement (20) dans sa position d'enserrement ;
- ouverture du volet d'enserrement (20) pour le passer dans sa position ouverte ;
- extraction du dispositif (4) à l'écart du patient par glissement de la région non adhérente du dispositif (4) située sous la paroi inférieure (18) entre la peau du patient et l'élément allongé (2), sans retourner l'élément allongé (2).

## Patentansprüche

1. Vorrichtung (4; 52) zum Befestigen eines länglichen Elements (2) an dem Körper eines Patienten, wobei das längliche Element (2) dazu bestimmt ist, teilweise in den Körper des Patienten eingeführt zu werden, die Vorrichtung umfassend:
- einen Rückhalter (6) des länglichen Elements (2), umfassend mindestens eine untere Wand (18), die zwischen dem länglichen Element (2) und dem Körper des Patienten platziert wird
- einen Klebestreifen (8), der den Rückhalter (6) trägt und dazu bestimmt ist, auf die Haut des Patienten aufgebracht zu werden, wobei der Klebestreifen (8) mindestens einen Bereich des der Befestigungsvorrichtung (4) definiert, der in Bezug auf die untere Wand (18) zum Ankleben an die Haut des Patienten versetzt ist
mindestens ein Bereich der Befestigungsvorrichtung (4), der sich unter der unteren Wand (18) entlang einer Achse (A-A') des Einführens des länglichen Elements (2) in den Rückhalter (6) befindet, dazu bestimmt ist, nicht an der Haut des Patienten zu kleben, wenn die Befestigungsvorrichtung (4) an der Haut des Patienten befestigt ist,
der Rückhalter (6) umfassend eine Umschließungsklappe (20), die in Bezug auf die untere Wand (18) zwischen einer offenen Position und einer Umschließungsposition des länglichen Elements (2), in der das längliche Element (2) dazu bestimmt ist, zwischen der unteren Wand (18) und der Umschließungsklappe (20) gehalten zu werden, gehandhabt werden kann.
**dadurch gekennzeichnet, dass** der gesamte Bereich der Befestigungsvorrichtung (4) unterhalb der unteren Wand (18) des Rückhalters (6) dazu bestimmt ist, nicht an der Haut des Patienten angeklebt zu sein.

2. Vorrichtung (4; 52) nach Anspruch 1, wobei die Umschließungsklappe (20) biegesteifer ist als das Klebeband (8).

3. Vorrichtung (4; 52) nach einem der Ansprüche 1 bis 2, wobei der Rückhalter (6) eine Befestigungsklappe (22) umfasst, die dazu geeignet ist, um auf die Umschließungsklappe (20) geklappt zu werden, um die Umschließungsklappe (20) in Position zu halten,
wobei die Befestigungslasche (22) vorteilhafterweise biegesteifer ist als das Klebeband (8).

4. Vorrichtung (4; 52) nach Anspruch 3, umfassend eine selektive Befestigungsanordnung (26) der Befestigungsklappe (22) an der Umschließungsklappe (20).

5. Vorrichtung (4; 52) nach einem der vorherigen Ansprüche, wobei der Rückhalter (6) mindestens eine Unterfütterunganordnung (24) des länglichen Elements (2) umfasst, die dazu bestimmt ist, in der Umschließungsposition zwischen der unteren Wand (18) und der Umschließungsklappe (20) eingefügt zu werden,
die Unterfütterunganordnung (24) umfassend vorteilhafterweise mindestens ein Element aus einem elastisch verformbaren Material (36), das von der Umschließungsklappe (20) und/oder der unteren Wand (18) hervorsteht.

6. Vorrichtung (4) nach Anspruch 5, wobei die Unterfütterunganordnung (24) einen ersten Satz von Stiften (48), die von der Klemmklappe (20) hervorstehen, und einen zweiten Satz von Stiften (50), die von der unteren Wand (18) hervorstehen, umfasst.

7. Vorrichtung (4; 52) nach einem der vorherigen Ansprüche, wobei der Klebestreifen (8) mindestens einen klebenden äußeren Bereich (44) aufweist, der dazu bestimmt ist, mit dem Körper des Patienten in Kontakt zu kommen, wobei der oder jeder klebende äußere Bereich (44) in Bezug auf den Rückhalter (6) und vorteilhafterweise in Bezug auf einen Austrittspunkt (17) des länglichen Teils (2) aus dem Patienten versetzt ist.

8. Vorrichtung (4; 52) nach einem der vorherigen Ansprüche, wobei der Klebestreifen (8) mindestens zwei äußere Klebebereiche (44) aufweist, die sich in Bezug auf die Einschubachse auf beiden Seiten des Rückhalters (6) befinden.

9. Vorrichtung (4) nach einem der vorherigen Ansprüche, wobei der Klebestreifen (8) eine untere Fläche (40) und eine obere Fläche (42) umfasst, wobei der Rückhalter (6) vorteilhafterweise durch Kleben an der oberen Fläche (42) befestigt ist.

10. Vorrichtung (52) nach einem der Ansprüche 1 bis 9, wobei der Klebestreifen (8) eine untere Fläche (40) und eine obere Fläche (42) umfasst, wobei mindestens ein mittlerer Bereich des Klebestreifens (8) an einer oberen Fläche der unteren Wand (18) befestigt ist, wobei der mittlere Bereich die untere Wand (18) überlappt.

11. Vorrichtung (52) nach Anspruch 10, wobei mindestens ein nicht gleitendes Kontaktelement (27) an der unteren Fläche der unteren Wand (18) befestigt ist.

12. Vorrichtung (52) nach einem der vorherigen Ansprüche, wobei der die Haltestütze (6) eine Absenkklappe (54) umfasst, wobei die Absenkklappe (54) auf den mittleren Bereich des Klebebands (8) aufgebracht ist.

13. Behandlungsset (1), umfassend eine Vorrichtung (4; 52) nach einem der vorherigen Ansprüche und ein längliches Element (2), das dazu bestimmt ist, teilweise in den Körper des Patienten eingeführt zu werden, wobei das längliche Element (2) vorzugsweise ausgewählt ist aus einem Katheter, insbesondere einem mittleren Katheter mit peripherischer Einführung, einem mittleren Venenkatheter und einem Dialysekatheter, einem Harnkatheter, einem nasogastrischen Katheter, einem Peritonealkatheter, einem Foley-Katheter, einem Drainagekatheter, einem Gallenkatheter, einem Nephrostomiekatheter, einem Gastrostomiekatheter, einem Epiduralkatheter, einem arteriellen Katheter, einem Pyloruskatheter, einem peri-nervösen Katheter und/oder einem Schlauch oder dem Körper einer Huber-Nadel

14. Behandlungsset (1) nach Anspruch 13, ferner umfassend einen oberen Verband (10), der dazu bestimmt ist, die Vorrichtung (4; 52) abzudecken, der obere Verband (10) umfassend eine Basislage (49), die die Vorrichtung (4) abdeckt, und eine Halteklappe (51) umfasst, die geeignet ist, um auf die Basislage (49) geklappt zu werden und einen Raum zum Festhalten eines Endes (53) des länglichen Elements (2) definiert.

15. Verfahren zum Befestigen eines länglichen Elements (2) an dem Körper eines Patienten, das nach teilweisem Einführen des länglichen Elements (2) in den Körper des Patienten durchgeführt wird, das Verfahren umfassend die folgenden Schritte:
- Bereitstellen einer Vorrichtung (4; 52) nach einem der Ansprüche 1 bis 12;
- Platzieren der Umschließungsklappe (20) in ihre geöffnete Position und Einsetzen des länglichen Elements (2) auf die untere Wand (18), indem die untere Wand (18) unter das längliche Element (2) geschoben wird, ohne das längliche Element (2) umzudrehen;
- Verstellen der Umschließungsklappe (20) in ihre Umschließungsposition;
- Befestigen mindestens eines äußeren Bereichs des Klebestreifens (8) an der Haut des Patienten, wobei mindestens ein Bereich der Vorrichtung (4; 52), der sich unter der unteren Wand (18) entlang einer Einführachse des länglichen Elements in den Halter befindet, nicht an der Haut des Patienten klebt.

16. Verfahren nach Anspruch 15, umfassend die folgenden Schritte:
- Ablösen von jedem äußeren Bereich (44) weg von der Haut des Patienten, wodurch die Umschließungsklappe (20) in ihrer Umschließungsposition gehalten wird;
- Öffnen der Umschließungsklappe (20), um sie in ihre offene Position zu verstellen;
- Entfernen der Vorrichtung (4) weg von dem Patienten, indem der nicht klebende Bereich der Vorrichtung (4) unter die untere Wand (18) zwischen der Haut des Patienten und dem länglichen Element (2) gebracht wird, ohne das längliche Element (2) zu drehen.

## Claims

1. A device (4; 52) for securing an elongated element (2) on the body of a patient, the elongated element (2) being intended to be partly inserted into the body of the patient, the device including:
- a support (6) for retaining the elongated element (2), including at least one lower wall (18) intended to be placed between the elongated element (2) and the body of the patient
- an adhesive strip (8) bearing the retaining support (6), and intended to be applied on the skin of the patient, the adhesive strip (8) defining at least one shifted region of the attachment device (4) with respect to the lower wall (18) intended to adhere to the skin of the patient
at least one region of the attachment device (4) located under the lower wall (18) along an axis (A-A') for inserting the elongated element (2) into the retaining support (6) is intended to be non-adherent to the skin of the patient when the attachment device (4) is secured onto the skin of the patient,
the retaining support (6) comprising a clasping flap (20) able to be maneuvered with respect to the lower wall (18) between an open position and a clasping position of the elongated element (2), in which the elongated element (2) is intended to be maintained between the lower wall (18) and the clasping flap (20),
**characterized in that** the entire region of the attachment device (4) located under the lower wall (18) of the retaining support (6) is intended to be non-adherent to the skin of the patient.

2. The device (4; 52) according to claim 1, **characterized in that** the clasping flap (20) is stiffer in flexure than the adhesive strip (8).

3. The device (4; 52) according to one of claims 1 or 2, **characterized in that** the retaining support (6) comprises an attachment flap (22), able to be folded back onto the clasping flap (20) in order to maintain the clasping flap (20) in position,
the attachment flap (22) being advantageously stiffer in flexure than the adhesive strip (8).

4. The device (4; 52) according to claim 3, **characterized in that** it comprises an assembly (26) for selectively attaching the attachment flap (22) on the clasping flap (20).

5. The device (4; 52) according to any of the preceding claims, **characterized in that** the retaining support (6) comprises at least an assembly (24) for blocking the elongated element (2), intended to be interposed between the lower wall (18) and the clasping flap (20) in the clasping position,
the blocking assembly (24) including at least one element in an elastically deformable material (36) protruding from the clasping flap (20) and/or of the lower wall (18).

6. The device (4), according to claim 5, **characterized in that** the blocking assembly (24) comprises a first set of pins (48) protruding from the clasping flap (20) and a second set of pins (50) protruding from the lower wall (18).

7. The device (4; 52) according to any of the preceding claims, **characterized in that** the adhesive strip (8) has at least one external adhesive region (44) intended to come into contact with the body of the patient, said or each external adhesive region (44) being shifted with respect to the retaining support (6) and advantageously with respect to an exit point (17) of the elongated element (2) out of the patient.

8. The device (4; 52) according to any of the preceding claims, **characterized in that** the adhesive strip (8) comprises at least two external adhesive regions (44) located on either side of the retaining support (6) with respect to the insertion axis.

9. The device (4) according to any of the preceding claims, **characterized in that** the adhesive strip (8) includes a lower surface (40) and an upper surface (42), the retaining support (6) being secured on the upper surface (42), advantageously by adhesive bonding.

10. The device (52) according to any of claims 1 to 9, **characterized in that** the adhesive strip (8) includes a lower surface (40) and an upper surface (42), at least one central region of the adhesive strip (8) being secured on an upper surface of the lower wall (18), the central region overlapping the lower wall (18).

11. The device (52) according to claim 10, **characterized in that** at least one non-sliding contact element (27) is secured on the lower surface of the lower wall (18).

12. The device (52) according to any of the preceding claims, **characterized in that** the retaining support (6) includes a folding-back flap (54), the folding-back flap (54) being applied on the central region of the adhesive strip (8).

13. A treatment kit (1) comprising a device (4; 52) according to any of the preceding claims and an elongated element (2) intended to be partly inserted into the body of the patient, the elongated element (2) being advantageously selected from a catheter, notably a central catheter with peripheral insertion, a central venous catheter, a dialysis catheter, a urinary catheter, a nasogastric probe, a peritoneal catheter, a Foley catheter, a draining catheter, a biliary probe, a nephrostomy probe, a gastrostomy probe, an epidural catheter, an arterial catheter, a urinary drainage probe, a perinerval catheter and/or a tube or the body of a Huber needle.

14. The treatment kit (1) according to claim 13, **characterized in that** it further comprises an upper bandage (10) intended to cover the device (4; 52), the upper bandage (10) comprising a base sheet (49) capping the device (4) and a maintaining flap (51) able to be folded back onto the base sheet (49) and defining a space for immobilizing one end (53) of the elongated element (2).

15. A method for securing an elongated element (2) on the body of a patient, applied after partial insertion of the elongated element (2) into the body of the patient, the method comprising the following steps:
- providing a device (4; 52) according to claims 1 to 12;
- placing the clasping flap (20) in its open position and inserting the elongated element (2) on the lower wall (18) by sliding the lower wall (18) under the elongated element (2), without turning the elongated element (2) over;
- having the clasping flap (20) passed into its clamping position;
- securing at least one external region of the adhesive strip (8) on the skin of the patient, at least one region of the device (4; 52) located under the lower wall (18) along an axis for inserting the elongated element in the support being non-adherent to the skin of the patient.

16. The method according to claim 15, **characterized in that** it includes the following steps:
- detaching each external region (44) away from the skin of the patient, by maintaining the clasping flap (20) in its clasping position;
- opening the clasping flap (20) for having it passed into its open position;
- extracting the device (4) away from the patient by sliding the non-adherent region of the device (4) located under the lower wall (18) between the skin of the patient and the elongated element (2), without turning over the elongated element (2).
